# FASCICULE DE BREVET EUROPEEN

(11) **EP 2 538 933 B1**
(45) Date de publication et mention de la délivrance du brevet: **19.10.2016**
(21) Numéro de dépôt: 11713498.1
(22) Date de dépôt: 28.02.2011
(51) Int. Cl.: A61K 31/00, A61K 36/54, A61K 36/53, A61P 17/12, A61P 35/00, A61K 31/05, A61K 31/045

(54) **L'HUILE ESSENTIELLE D'ORIGAN POUR LE TRAITEMENT DES KERATOSES CANCÉREUSES**
ÄTHERISCHES ÖL AUS OREGANO FÜR DIE BEHANDLUNG VON MALIGNER KERATOSE
ESSENTIAL OIL OF OREGANO FOR THE TREATMENT OF MALIGN KERATOSIS

(30) Priorité: 26.03.2010 US 282751 P; 26.02.2010 FR 1000800
(43) Date de publication de la demande: 02.01.2013
(73) Titulaire: L'Oréal, 75008 Paris (FR); Institut Curie, 75248 Paris Cedex 05 (FR); Centre National de la Recherche Scientifique, 75794 Paris Cedex 16 (FR)
(72) Inventeur: MARROT, Laurent, F-93190 Livry Gargan (FR); SOEUR, Jérémie, F-92340 Bourg La Reine (FR); HUANG, Meng-Er, F-91400 Orsay (FR)
(74) Mandataire: Sellin, Carole
(86) Numéro de dépôt international: PCT/FR2011/050411
(87) Numéro de publication internationale: WO 2011/104490

(56) Documents cités:
- FR-A1- 2 830 198
- US-A1- 2008 213 410
- US-A1- 2008 233 218
- KAUR MANJINDER ET AL: "Skin cancer chemopreventive agent, alpha-santalol, induces apoptotic death of human epidermoid carcinoma A431 cells via caspase activation together with dissipation of mitochondrial membrane potential and cytochrome c release", CARCINOGENESIS, vol. 26, no. 2, février 2005 (2005-02), pages 369-380, XP002593567, OXFORD ISSN: 0143-3334 cité dans la demande
- DWIVEDI CHANDRADHAR ET AL: "Chemopreventive effects of alpha-santalol on skin tumor development in CD-1 and SENCAR mice.", CANCER EPIDEMIOLOGY BIOMARKERS AND PREVENTION, vol. 12, no. 2, février 2003 (2003-02), pages 151-156, XP002593568, ISSN: 1055-9965
- DWIVEDI C ET AL: "CHEMOPREVENTIVE EFFECTS OF SANDALWOOD OIL ON SKIN PAPILLOMAS IN MICE", EUROPEAN JOURNAL OF CANCER PREVENTION, vol. 6, no. 4, 1 août 1997 (1997-08-01), pages 399-401, XP001121309, LIPPINCOTT WILLIAMS & WILKINS, PHILADELPHIA, US ISSN: 0959-8278
- KOBA KOFFI ET AL: "In vitro cytotoxic activity of Cymbopogon citratus L. and Cymbopogon nardus L. essential oils from Togo", BANGLADESH JOURNAL OF PHARMACOLOGY, vol. 4, no. 1, 2009, pages 29-34 URL, XP002593569, cité dans la demande
- KOBA K ET AL: "Chemical composition and in vitro cytotoxicactivity of essential oils from two tropical Lamiaceae:Aeollanthus pubescens Benth. and Ocimum gratissimum L", JOURNAL OF ESSENTIAL OIL-BEARING PLANTS, vol. 10, no. 1, 2007, pages 60-69, XP009136539, BHALLA, DEHRA DUN, IN ISSN: 0972-060X
- anonyme: "Personal, Body, Care, Baby, Care, Cosmetics - Ingredients Used", , 11 mai 2009 (2009-05-11), pages 52-106, XP002593572, Extrait de l'Internet: URL:http://www.earthorigin.co.za/ingredien ts.html [extrait le 2010-07-22]
- Anonymous: "Price List and Order Form", , 22 mars 2008 (2008-03-22), pages 1-6, XP002593573, Extrait de l'Internet: URL:http://web.archive.org/web/20080322083 726/http://www.thevictoriangarden.co.za/Pr iceOrder.html [extrait le 2010-07-22]
- Anonymous: "Welcome to the Victorian Garden of the 1800s...", , 25 juin 2010 (2010-06-25), pages 1-8, XP002593574, Extrait de l'Internet: URL:http://webcache.googleusercontent.com/ search?q=cache:zOdC1BbW9d0J:www.thevictori angarden.co.za/AI_FacialProducts_pop-up.ht m+site:http://www.thevictoriangarden.co.za /AI_FacialProducts_pop-up.htm&cd=1&hl=en&c t=clnk&gl=de&tbo=1 [extrait le 2010-07-22]
- anonyme: "Avocado Oil", , 14 juin 2008 (2008-06-14), pages 1-2, XP002593575, Extrait de l'Internet: URL:http://web.archive.org/web/20080614124 629/http://www.naturesgift.com/carrier_oil s/avocadoOil.htm [extrait le 2010-07-22]
- drbeckl2: "Skin Cancer Forum - wild oregano oil - skin cancer testimonies", , 26 mai 2008 (2008-05-26), pages 1-6, XP002593576, Extrait de l'Internet: URL:http://www.topicalinfo.org/forum/topic .asp?TOPIC_ID=308 [extrait le 2010-07-23]
- Anonymous: "Testimonials", , 6 août 2007 (2007-08-06), pages 1-7, XP002593577, Extrait de l'Internet: URL:http://web.archive.org/web/20070806205 829/http://www.wildoiloforegano.com/index. php?page=testimonials [extrait le 2010-07-23]
- Anonymous: "FAQ", , 6 août 2007 (2007-08-06), pages 1-7, XP002593578, Extrait de l'Internet: URL:http://web.archive.org/web/20070806205 742/http://www.wildoiloforegano.com/index. php?page=faq [extrait le 2010-07-23]
- TANG XIUWEI ET AL: "CP-31398 restores mutant p53 tumor suppressor function and inhibits UVB-induced skin carcinogenesis in mice", JOURNAL OF CLINICAL INVESTIGATION, vol. 117, no. 12, décembre 2007 (2007-12), pages 3753-3764, XP002593795, ISSN: 0021-9738
- QEINSPAHR JANINE ET AL: "Expression of p53 protein in actinic keratosis, adjacent, normal-appearing, and non-sun-exposed human skin", CANCER EPIDEMIOLOGY BIOMARKERS AND PREVENTION, vol. 6, no. 8, 1997, pages 583-587, XP002593796, ISSN: 1055-9965
- ANONYME: "Welcome to Pure Healing Europe: Herpes Treatments and Warts Treatments", INTERNET CITATION, [Online] 21 novembre 2009 (2009-11-21), pages 1-6, XP002593797, Extrait de l'Internet: URL:http://www.purehealing-eu.com/body-war ts/treatment-body-warts.ht> [extrait le 2010-07-20]
- J-P. ORTONNE: "From actinic keratosis to squamous cell carcinoma", BRITISH JOURNAL OF DERMATOLOGY, vol. 146, no. s61, 1 avril 2002 (2002-04-01), pages 20-23, XP055007540, ISSN: 0007-0963, DOI: 10.1046/j.1365-2133.146.s61.6.x cité dans la demande
- Anonymous: "Wild Oil of Oregano", , 17 janvier 2010 (2010-01-17), page 1, XP055007479, Extrait de l'Internet: URL:http://web.archive.org/web/20100117235 421/http://www.oiloforegano.com/oil-of-ore gano-research-article-3.html [extrait le 2011-09-19]
- Erhardt W, Götz E, Bödecker N, Seybold S: "Zander - Handwörterbuch der Pflanzennamen - 16. Auflage", 2000, Eugen Ulmer, Stuttgart, XP002659476, ISBN: 3-8001-5080-8 pages 556-557, page 556
- S Bouhdid ET AL: "Antibacterial and antioxidant activities of Origanum compactum essential oil", African Journal of Biotechnology, 16 mai 2008 (2008-05-16), pages 1563-1570, XP055007547, Extrait de l'Internet: URL:http://www.academicjournals.org/AJB/PD F/pdf2008/16May/Bouhdid et al.pdf [extrait le 2011-09-20]
- Mark Naylor: "Cutaneous Manifestations of Human Papilloma Virus", , 5 March 2009 (2009-03-05), pages 1-31, XP055162826, Retrieved from the Internet: URL:http://web.archive.org/web/20090305073 730/http://www.telemedicine.org/warts/cutm anhpv.htm [retrieved on 2015-01-16]
- G. L. Knight ET AL: "Cooperation between Different Forms of the Human Papillomavirus Type 1 E4 Protein To Block Cell Cycle Progression and Cellular DNA Synthesis", Journal of Virology, vol. 78, no. 24, 15 December 2004 (2004-12-15), pages 13920-13933, XP055162830, ISSN: 0022-538X, DOI: 10.1128/JVI.78.24.13920-13933.2004

## Description

La présente invention est dans le domaine de la prévention et du traitement thérapeutique ciblé des kératoses actiniques.

La lumière solaire est une cause avérée du développement de cancers cutanés, en particulier de carcinomes spinocellulaires (SCC) et basocellulaires (BCC) impliquant les kératinocytes (Cleaver, J.E., et al. 2002). Le domaine ultraviolet (300-400 nm) est majoritairement impliqué dans ce processus, avec un impact prépondérant des UVB (300-320 nm), même si les UVA contribuent à l'augmentation du risque. Il est clairement établi dans la littérature que les UV solaires induisent des lésions dans l'ADN du génome des cellules épidermiques, en particulier des dimères de pyrimidine. Ces dommages, s'ils ne sont pas réparés correctement, sont une source de mutations qui constituent la première étape vers la tumorigenèse (Marrot, L. et al. 2008).

La protéine p53 joue un rôle très important dans l'homéostasie cutanée, en particulier au niveau du contrôle du cycle cellulaire. Lorsque des lésions à l'ADN sont induites, p53 est modifiée par diverses phosphorylations (principalement sur la sérine 15) qui l'activent comme facteur de transcription. Si le niveau d'endommagement est acceptable, p53 permet l'arrêt du cycle cellulaire en phase G0/G1 et stimule les fonctions de réparation de l'ADN. Par contre, si le matériel génomique est trop altéré, p53 favorise l'enclenchement de l'apoptose, éliminant ainsi les cellules à risque (Kulms, D. et al. 2000).

Le gène codant pour la protéine p53 est couramment endommagé par les UV et, dans certains cas, subit des mutations, notamment des mutations CC vers TT. Les cellules ainsi mutées peuvent perdre la réponse spécifique aux UV contrôlée par la protéine p53 : la réparation est alors moins efficace et la présence de dégâts à l'ADN n'entraîne plus d'arrêt de prolifération : la réplication peut se dérouler en présence de lésions, augmentant ainsi le risque d'erreurs (mutations) et entraînant, à terme, une forte instabilité génétique. Comme les cellules mutées entrent moins facilement dans le processus d'apoptose, elles finissent par manifester un avantage de croissance par rapport aux cellules normales. Ce processus d'expansion clonale de cellules anormales crée des patches précancéreux dans les organes atteints (Brash, D. E. 2006).

L'apoptose est un processus complexe qui peut avoir des origines diverses, mais il est aujourd'hui établi que la mitochondrie y joue un rôle important. Différents signaux moléculaires, comme par exemple, l'équilibre Bcl2/Bax avec le possible concours de p53, sont capables de modifier la perméabilité de la membrane mitochondriale et de favoriser le relargage de cytochrome C dans le cytosol. Ce processus peut initier l'activation de la voie des caspases, enzymes en charge de la dégradation de certains composés intracellulaires, qui conduit de façon irréversible à l'apoptose (Gogvadze et al., 2008). Par ailleurs, il a aussi été montré que la perméabilisation de la membrane mitochondriale permettait le déversement de différentes molécules mitochondriales pro-oxydantes dans le cytosol, permettant l'induction de l'apoptose. Dans le cas de la peau, il a été montré que les kératoses et les carcinomes étaient souvent constitués de kératinocytes porteurs de mutation dans le gène p53 (Taguchi, M. et al. 1994). De plus, l'analyse des séquences où se produisent ces mutations montre qu'elles correspondent très fréquemment à des sites favorables à la formation des dimères de pyrimidine.

Au plan dermatologique, les kératoses apparaissent comme des zones de dysplasie épithéliale que l'on rencontre dans les régions du corps fréquemment exposées au soleil, et sont souvent associées au photo-vieillissement. Entre 1% et 10% de ces kératoses peuvent évoluer vers une cancérisation, avec développement de carcinomes squameux SCC, les autres régressent spontanément (Ortonne, J. P. 2002).

Pour étudier les mécanismes de cancérisation cutanée, il est possible d'utiliser des kératinocytes portant des mutations dans le gène p53. C'est le cas de la lignée HaCat qui a été initialement décrite par Fusenig et al. (Fusenig, N. E. et al. 1998) et qui est aujourd'hui très fréquemment mise en oeuvre dans différents laboratoires de recherche en dermatologie. Ainsi par exemple, des cellules HaCat ont été intégrées dans un modèle organotypique de peau en présence de kératinocytes normaux : dans un tel système, il a été possible de mimer les effets promoteurs de l'exposition solaire en montrant la capacité des UVB à stimuler l'expansion clonale des cellules HaCat (Mudgil, A. V. et al. 2003) qui sont mutées en p53. Plus récemment, il a été rapporté que des cellules HaCat exposées aux UVA et réimplantées dans des souris pouvaient être à l'origine de tumeurs cutanées (Wischermann, K. et al. 2008). Ces données suggèrent que des produits induisant une cytotoxicité significativement plus intense dans des cellules HaCat par rapport à des kératinocytes humains normaux (ou à des lignées kératinocytaires non mutées en p53) seraient potentiellement capables de traiter les kératoses actiniques et également de réduire le risque de développement des carcinomes, notamment SCC. C'est pour cette raison que les travaux décrits ci-après ont consisté à comparer et comprendre l'effet cytotoxique d'huiles essentielles sur des kératinocytes humains normaux, sur des kératinocytes HaCat (porteurs d'une mutation en p53) et sur des kératinocytes immortalisés issus de carcinome.

Les huiles essentielles (HE) et leurs produits dérivés, rencontrent actuellement un succès important auprès des consommateurs. Cet attrait tout particulier pour les produits d'origine naturelle (agroalimentaire, cosmétologie, pharmacologie...) ou issus du concept de naturalité, est la conséquence d'une demande générale de produits issus d'une production de type développement durable (chimie verte, agriculture bio). Cet intérêt pour la naturalité et ses produits de médecine douce (aromathérapie, phytothérapie) trouve également son origine dans les limites atteintes par des traitements de la médecine classique (résistances virales, bactériennes, traitement contre le cancer...). L'absence d'effets secondaires trop importants, dans le cadre de traitements aromathérapeutiques bien encadrés et raisonnés est un atout majeur pour l'utilisation des huiles essentielles.

Les huiles essentielles sont des produits volatiles complexes possédant une puissante odeur, caractéristique de la partie de la plante utilisée pour sa fabrication. Sur environ 800 000 espèces végétales répertoriées, seules les plantes aromatiques sont utilisées pour l'obtention d'huiles essentielles. Il s'agit de plantes possédant suffisamment de cellules synthétisant et sécrétant ces molécules aromatiques, soit environ 3000 plantes d'intérêt biologique. Environ 300 huiles essentielles représentent la majeure partie des huiles essentielles commercialisées.

Les huiles essentielles correspondent à un mélange complexe de métabolites secondaires (molécules non essentielles à la survie de la plante) synthétisés et sécrétés par des organes spécialisés : les poils glandulaires épidermiques, les poches et canaux glandulaires (schizogènes ou schizolysigènes). Ces métabolites secondaires sont représentés par une très grande diversité de molécules chimiques. Les plus courants sont les terpènes (mono-, sesquiet diterpène : C10, C15 et C20 respectivement) et autres molécules aromatiques (molécules cycliques). Toutes les fonctions chimiques sont présentes dans les huiles essentielles: aldéhydes, cétones, alcools, peroxydes, lactones, éthers, esters...

Les huiles essentielles sont obtenues après hydro-distillation du matériel végétal de la plante aromatique. Aujourd'hui différentes méthodes d'extractions sont utilisées, parmi lesquelles on peut citer l'extraction au CO₂ supercritique et l'extraction par solvants.

Les huiles essentielles possèdent un potentiel cosmétique et thérapeutique reconnu, et sont principalement utilisées pour leurs activités bactéricides, virucides, antioxydantes, antiinflammatoires, mais également pour leur caractère odorant, susceptible d'engendrer un sentiment de bien-être. Plusieurs modes d'utilisation des huiles essentielles sont possibles : inhalation, ingestion ou application cutanée. Les huiles essentielles pures ne sont quasiment jamais appliquées directement sur la peau, car elles sont souvent irritantes, mais diluées dans d'autres huiles végétales (huile d'olive, de tournesol...). Une application cutanée est réalisée dans le cadre de massages, de traitements locaux (infections) ou encore lors de l'utilisation de parfums (constituants majeurs).

Outre leurs intérêts cosmétiques, les huiles essentielles sont également utilisées dans le cadre de certains traitements antibiotiques, pour leur capacité à augmenter leur efficacité, ou encore pour lutter contre des infections des voies respiratoires. De la même façon, une proportion non négligeable (environ 35%) des patients européens atteints de cancer, dont les traitements classiques n'ont pu permettre la guérison, ont fait appel à des méthodes complémentaires et alternatives à la médecine classique (Molassiotis, A. et al. 2005), comme l'aromathérapie. Certaines études mettent en évidence des propriétés anticancéreuses et/ou anti-prolifératives des huiles essentielles vis-à-vis de certaines lignées cancéreuses. Ainsi, des travaux récents ont montré des activités potentiellement anti-carcinogènes ou antiprolifératives de certaines huiles essentielles sur des lignées de cellules leucémiques (Kumar, A. et al. 2008), (Verma, M. et al. 2008), lignées de cancer du colon (Sharma, P. R. et al. 2008), cellules de cancer du sein (Diaz, C. et al. 2008), cellules de mélanome (Loizzo, M. R. et al. 2008).

D'autre travaux ont montré également une activité anti-proliférative de certains composés isolés des huiles essentielles, comme l'alpha santalol sur une lignée de carcinome épidermoïde (Kaur, M et al. 2005), ou encore celle du terpinène-4-ol de l'huile essentielle de l'arbre à thé sur des cellules de mélanomes (Calcabrini, A. et al. 2004).

Concernant les kératinocytes, la cytotoxicité de certaines HE a été étudiée vis-à-vis de la lignée kératinocytaire HaCat mutée en p53 (Koba K. et al. 2009) ou de la lignée kératinocytaire SVK14 (Itharat, A. et al. 2004).

La demande US2008213410 divulgue une composition comprenant de l'huile essentielle d'origan qui est utilisée pour traiter les verrues.

L'invention concerne une huile essentielle extraite de plantes du genre Origanum, ou une composition comprenant ladite huile, pour une utilisation thérapeutique chez l'être humain dans la prévention, ou le traitement ciblé, de kératoses en phase de transformation cancéreuse, de kératinocytes cancéreux, ou de carcinome provenant de la transformation de kératoses, lesdites kératoses, kératinocytes cancéreux ou carcinome, se caractérisant par des cellules porteuses de mutations dans le gène p53.

L'invention concerne plus particulièrement l'huile essentielle extraite des différentes parties (racines, tiges, écorce, feuilles) *d'Origanum compactum* .

En effet, de façon surprenante, les présents inventeurs ont montré que l'huile essentielle d'Origan compact *(Origanum compactum,* famille des Lamiacées) et l'huile essentielle de Bois de rose *(Aniba rosaeodora,* famille des Lauracées), peuvent induire la mort cellulaire par apoptose de façon ciblée chez des kératinocytes humains cancéreux et mutés en p53 précancéreux par rapport aux kératinocytes normaux. Les inventeurs ont également montré que l'effet cytotoxique ciblé est présent avec les constituants prépondérants de ces huiles essentielles, même en l'absence des autres constituants.

Ce traitement a en outre l'avantage de ne pas générer d'inflammation, l'apoptose n'étant pas un processus pro-inflammatoire.

L'invention concerne plus spécifiquement différentes applications thérapeutiques de compositions comprenant de l'huile essentielle *d'Origanum compactum* dans la prévention ou le traitement de kératoses, plus particulièrement des kératoses en phase de transformation, précancéreuses, ou bien cancéreuses, notamment des carcinomes cutanés, de préférence UV-induits. Les différentes applications thérapeutiques selon l'invention sont préférentiellement pour l'être humain.

Les applications prophylactiques ou thérapeutiques au sens de la présente invention s'entendent de traitements visant à préserver ou restaurer la santé du patient, et non de traitements à pure visée esthétique ou cosmétique.

Dans le cadre de cette invention, les compositions considérées sont pour une utilisation dans le traitement ou la prévention des kératoses en phase de transformation. De telles kératoses à traiter contiennent des cellules tumorales ou bien des cellules potentiellement tumorales. Dans cette situation en effet, un médecin recommanderait l'excision des kératoses pour des raisons thérapeutiques, étant donné l'évolution probable ou certaine vers un carcinome. Les kératoses considérées sont de préférence de taille importante, elles présentent notamment un important risque de cancérisation à court ou moyen terme.

La présente invention concerne également des compositions pour une utilisation dans le traitement ou la prévention de l'apparition de kératinocytes cancéreux ou de carcinome provenant de la transformation de kératoses, notamment des carcinomes cutanés UV-induits.

Par kératose (ou kératodermie ou hyperkératose) on entend une hyperplasie de la couche cornée (Stratum corneum) épidermique. Par kératose actinique ou kératose solaire, il est fait référence à une kératose induite par l'exposition au rayonnement solaire. Dans le cadre de la présente invention, des kératoses tout particulièrement préférées pour les applications thérapeutiques, sont des kératoses actiniques. De préférence, les kératoses considérées sont constituées ou comprennent majoritairement des cellules mutées en p53.

Du fait de leur multiplication ces dernières décennies, il existe en effet un fort besoin de prévention et / ou de traitement des kératoses en phase de transformation, présentant un danger de cancérisation, et des carcinomes issus de telles kératoses.

Par prévention des kératoses en phase de transformation, des kératinocytes précancéreux ou cancéreux, ou des carcinomes provenant de la transformation de kératoses, on entend notamment l'obtention d'un effet préventif tel que les kératoses ne se transforment pas, ou n'évoluent pas vers un stade cancéreux.

Par traitement des kératoses en phase de transformation, des kératinocytes cancéreux ou des carcinomes provenant de la transformation de kératoses, on entend notamment un effet tel que les kératoses cancéreuses ou bien en phase de transformation, déjà existantes, stoppent leur croissance et régressent, voire disparaissent entièrement ou bien retournent à un stade non cancéreux, ou bien le nombre de cellules cancéreuses ou en phase de transformation diminue. La prévention ou le traitement des kératoses est particulièrement approprié dans le cas de peaux ayant été soumises à de fortes expositions aux UV ou des expositions répétées aux UV. Ces situations sont en effet susceptibles de produire des mutations dans p53, le développement de kératoses et leur évolution vers un stade cancéreux.

La prévention ou le traitement des kératoses cancéreuses ou en phase de transformation, dans une application thérapeutique selon l'invention, est également particulièrement approprié dans le cas de kératoses en phase de développement, c'est-à-dire que les kératoses sont déjà présentes sur la peau et qu'elles croissent. Au cours de ce processus, le risque d'évolution vers un stade cancéreux est en effet accrû. Dans une telle situation, les compositions pour l'utilisation thérapeutique selon l'invention permettent de réduire le risque de transformation vers un stade cancéreux.

L'un des avantages majeurs des compositions, pour l'utilisation en thérapie décrite, réside dans l'action ciblée des huiles essentielles *d'Origanum compactum* En effet, l'huile essentielle *d'Origanum compactum* et celle *d'Aniba rosaeodora,* ou au moins l'un de leurs constituants, présentent, dans une fenêtre de concentration, une action ciblée à l'encontre des cellules précancéreuses et cancéreuses (mutées en p53), qui peuvent être hyperprolifératives ; les cellules normales sont quant à elles peu affectées par la cytotoxicité. Il est donc possible de traiter l'ensemble de la peau d'un individu sans aucun effet délétère pour les kératinocytes normaux. Cette qualité est particulièrement appréciable dans le cadre d'un traitement, car elle permet d'éviter les effets secondaires liés à la thérapie ; en effet la toxicité de ce traitement vis-à-vis des cellules normales, notamment des kératinocytes normaux, environnant les zones à traiter, est faible.

Il est à noter que les inventeurs sont en effet les premiers à avoir réalisé une étude comparée des impacts des huiles essentielles sur des kératinocytes mutés en p53 et sur des kératinocytes normaux. Aucune donnée relative à une telle comparaison de cytotoxicité n'avait été obtenue avant la présente invention. L'action ciblée des huiles essentielles et de leurs constituants n'avait donc jamais été mise en lumière avant l'invention. Au vu de ce qui précède, la présente invention permet donc une prévention ou un traitement ciblé des kératoses cancéreuses ou en phase de transformation.

Par cytotoxicité ciblée au sens de l'invention, on entend que la propriété de cytotoxicité est préférentiellement induite dans les cellules hyperprolifératives et/ou mutées en p53 (par exemple HaCat et A431) par rapport aux cellules non hyperprolifératives, non mutées en p53 (par exemple HEK001 et NHEK894), c'est-à-dire que les taux de toxicité sont au moins 1,5 fois plus élevés dans les cellules mutées que dans les cellules non mutées, de préférence au moins 2 fois plus élevés. De préférence, la viabilité des cellules non hyperprolifératives, non mutées reste de l'ordre de 40% au moins, voire de 50%, de préférence de 60%. Les inventeurs ont en effet mis en évidence l'existence de gammes de concentrations auxquelles une telle cytotoxicité ciblée pouvait être obtenue, épargnant les cellules saines, notamment les kératinocytes normaux non cancéreux.

Cette action ciblée permet également d'envisager des applications répétées, du fait que les compositions sont sans danger pour les cellules saines.

Dans les compositions selon la présente divulgation l'huile essentielle considérée, ou le constituant d'huile essentielle considéré, constitue le principe actif de la composition, ou l'un des principes actifs, du fait que les inventeurs ont mis en évidence la cytotoxicité ciblée des huiles essentielles et de leurs constituants à l'encontre de kératinocytes mutés. Selon une mise en oeuvre préférée de la présente divulgation, la composition contient au moins un des constituants de l'huile essentielle *d'Origanum compactum* ou *d'Aniba rosaeodora,* de préférence elle comprend au moins 10%, ou au moins 15 ou 20% en masse de linalol, de carvacrol ou de thymol. De préférence il s'agit d'une composition comprenant une huile essentielle ou un mélange d'huiles essentielles, tel que le mélange comprenne lesdites proportions de linalol, de carvacrol ou de thymol. L'invention concerne également des compositions où lesdits pourcentages correspondent à des pourcentages chromatographiques. En effet, dans la section expérimentale, la cytotoxicité de deux huiles essentielles, l'huile essentielle d'Origan compact *(Origanum compactum,* famille des Lamiacées) et l'huile essentielle de Bois de rose *(Aniba rosaeodora,* famille des Lauracées), a été étudiée sur des cellules épidermiques humaines : la lignée kératinocytaire HaCat (mutée en p53 et immortalisée spontanément), la lignée A431 (issue d'un carcinome spino-cellulaire) ainsi que sur des kératinocytes épidermiques primaires normaux humains.

L'huile essentielle d'Origan compact se compose majoritairement de deux monophénols (terpénoïde) : le thymol et le carvacrol. Cette huile essentielle est préconisée dans le cadre de traitement de colites, de désordres pulmonaires. Elle permet de lutter contre la fatigue mais est surtout un puissant bactéricide à large spectre. Elle est irritante et hépato-toxique.

L'huile essentielle de Bois de rose se compose à 80% de linalol. Ses propriétés sont utilisées pour traiter les peaux irritées, fatiguées et ridées. Elle est également anti-infectieuse (moins puissante que l'huile essentielle d'Origan), immunostimulante et tend améliorer les comportements dépressifs. Elle est très faiblement irritante et faiblement toxique.

La présente divulgation concerne préférentiellement des compositions comprenant de l'huile essentielle *d'Origanum compactum* et/ou *d'Aniba rosaeodora* ou au moins l'un de leurs constituants majoritaires choisis parmi le linalol, le thymol et le carvacrol.

Bien que l'activité cytotoxique d'un constituant isolé soit moindre que celle d'une huile essentielle complète, selon une autre mise en oeuvre de la divulgation, la composition comprend au moins un constituant de l'huile essentielle *d'Origanum compactum* ou *d'Aniba rosaeodora,* par exemple elle comprend une huile essentielle contenant au moins un constituant de l'huile essentielle *d'Origanum compactum* ou *d'Aniba rosaeodora.*

Les différents constituants des huiles essentielles sont bien connus de l'homme du métier et peuvent être obtenus auprès des sociétés commercialisant lesdites huiles essentielles. L'exemple 1 de la présente demande fournit une caractérisation analytique des différents constituants détectés dans les huiles essentielles *d'Origanum compactum* et *d'Aniba rosaeodora.* Il est à noter qu'aucune de ces deux huiles essentielles ne contient d'alpha-santalol.

Il est tout particulièrement préféré, dans le cadre de la présente invention, que le constituant d'une des deux huiles essentielles citées, dont il est fait usage ne soit pas l'oxyde de linalol, de préférence même, ne soit pas un oxyde.

De préférence, le composant utilisé est un alcool ou un phénol, de manière plus particulièrement préférée un terpénol ou alcool terpénique, tel que le linalol, le terpinéol, le nérol, le géraniol, le citronellol, ou le menthanol, ou un terpénoïde tel que le citral, le menthol, le carvacrol ou le thymol, d'autres terpènes également envisagés sont les P-cymène, le terpinène, le myrcène et le caryophyllène béta. Des constituants tout particulièrement préférés sont le linalol, le carvacrol et le thymol.

Selon d'autres mises en oeuvre préférées des compositions pour une utilisation thérapeutique telle que décrite précédemment, le constituant est choisi parmi le 1,8-cinéole, le terpinolène, le linalol, l'alpha-terpinéol, le géraniol, l'alpha-copaène, l'alpha et le beta-selinène, le benzoate de benzyle, l'alpha-thujène, l'alpha pinène, le myrcène, l'alpha-phellandrène, l'alpha-terpinène, le paracymène, le beta-phellandrène, le gamma-terpinène, le thymol, le carvacrol et le beta-caryophyllène. Il s'agit en effet des constituants de l'une ou l'autre des deux huiles essentielles d'origan et de bois de rose, dont la proportion dans la partie volatile de l'huile est supérieure à 0,5% (en pourcentages chromatographiques).

Selon une autre mise en oeuvre des compositions pour l'application thérapeutique mentionnée, il est fait usage d'une huile essentielle comprenant au moins 0,5% en masse ou en pourcentages chromatographiques, de préférence au moins 1%, voire 5%, d'au moins l'un des composants suivants: le 1,8-cinéole, le terpinolène, le linalol, l'alpha-terpinéol, le géraniol, l'alpha-copaène, l'alpha et le beta-selinène, le benzoate de benzyle, l'alpha-thujène, l'alpha pinène, le myrcène, l'alpha-phellandrène, l'alpha-terpinène, le paracymène, le beta-phellandrène, le gamma-terpinène, le thymol, le carvacrol et le beta-caryophyllène. De préférence, il s'agit d'un pourcentage chromatographique, s'appliquant à la partie volatile de l'huile essentielle.

De manière tout particulièrement préférée, il est fait usage de compositions comprenant au moins un constituant d'huile essentielle choisi parmi les composants suivants : le linalol, le thymol et le carvacrol. Il s'agit en effet, pour le linalol, du composé majoritaire de l'huile essentielle de bois de rose, et pour le thymol et le carvacrol, des deux composés majoritaires de l'huile essentielle d'origan.

Selon une mise en oeuvre préférée, pour une utilisation en thérapie telle que décrite, l'invention concerne également une composition consistant ou comprenant une huile essentielle ayant au moins l'un des constituants suivants : linalol, thymol et carvacrol. De préférence, au moins l'un de ces constituants est présent, dans l'huile essentielle ou dans la composition, en proportion supérieure à 0,5%, de préférence supérieure à 1%, 5% voire 10%, 15% ou 20% en masse. Alternativement, il peut s'agir de pourcentages chromatographiques. Une telle huile essentielle est par exemple l'huile essentielle de thym, de serpolet ou de sarriette (taux important de thymol ou carvacrol) ou l'huile essentielle de basilic, de thym (à linalol, Thymus vulgaris linaloliferum), de lavande ou de bois de Shiu (nommé également bois de Hô).

La présente divulgation concerne tout particulièrement des compositions pour l'application thérapeutique mentionnée, comprenant au moins 10%, de préférence au moins 15% ou au moins 20% de l'un des constituant suivants : linalol, carvacrol et thymol. D'autres compositions également envisagées sont telles que la teneur cumulée de linalol, carvacrol et thymol est supérieure à 10%, de préférence supérieure à 15%, voire supérieure à 20% en masse. Alternativement, il peut s'agir de pourcentages chromatographiques.

Selon une autre mise en oeuvre de la divulgation, le composant d'huile essentielle *d'Origanum compactum* ou *d'Aniba rosaeodora,* entrant dans les compositions pour les applications thérapeutiques envisagées, est choisi parmi l'alpha pinène, le camphène, le myrcène, l'alpha terpinène, le paracymène, le trans-ocimène, le gamma-terpinène, le linalol, le terpinen-4-ol, le beta-caryophyllène, l'alpha-humulène et l'oxyde de caryophyllène. Il s'agit en effet des composants qui sont communs aux deux huiles essentielles d'origan et de bois de rose.

Selon une autre mise en oeuvre, la divulgation concerne également une composition comprenant une huile essentielle ayant au moins un constituant en commun avec l'huile essentielle de Bois de rose ou d'Origan compact, pour une utilisation thérapeutique dans le traitement des kératoses. De préférence, ledit composant est l'un des composants suivants : l'alpha pinène, le camphène, le myrcène, l'alpha terpinène, le paracymène, le trans-ocimène, le gamma-terpinène, le linalol, le terpinen-4-ol, le beta-caryophyllène, l'alpha-humulène et l'oxyde de caryophyllène. De préférence, au moins l'un des ces composants est présent en proportion supérieure à 0,5%, de préférence supérieure à 1%, 5% voire 10%, 15% ou 20% en masse. Alternativement, il peut s'agir de pourcentages chromatographiques.

Selon une autre mise en oeuvre divulguée dans le cadre de la présente invention, l'huile essentielle, ayant un constituant commun avec l'huile essentielle de Bois de rose ou d'Origan compact, est extraite de plantes de la famille des Lamiacées ou des Lauracées.

Plus particulièrement, l'huile essentielle ayant un constituant commun avec l'huile essentielle de Bois de rose ou d'Origan compact, est extraite de plantes provenant de l'un des genres botaniques suivants, de la famille des Lamiacées : *Origanum, Acinos, Agastache, Ajuga, Calamintha, Glechoma, Hyssopus, Lamium, Lavandula, Leonurus, Lycopus, Melissa, Melittis, Mentha, Micromeria, Monarda, Ocimum, Orthosiphon, Perilla, Perovskia, Phlomis, physostegia, Pogostemon, Prostanthera, Prunella, Rosmarinus, Salvia, Satureja, Scutellaria, Slderitis, Solenostemon, Stachys, Teucrium* et *Thymus.* De préférence, il s'agit du genre *Origanum.*

Selon une autre mise en oeuvre de la divulgation, il est fait usage d'une composition comprenant une huile essentielle, ayant un constituant commun avec l'huile essentielle de Bois de rose ou d'Origan compact, ou d'un constituant de l'huile essentielle de Bois de rose ou d'Origan, qui soit capable de générer une situation pro-oxydante endogène, qui se produise spécifiquement dans des kératinocytes mutés en p53. Des huiles essentielles ayant cette propriété sont notamment l'huile essentielle d'origan compact et l'huile essentielle de bois de rose. Des résultats en ce sens sont présentés dans l'exemple 2 de la partie expérimentale. L'analyse moléculaire détaillée dans la section expérimentale montre que ces huiles essentielles provoquent une déstabilisation de la membrane mitochondriale et un relargage d'espèces réactives de l'oxygène, à l'origine de la cytotoxicité ciblée aux cellules précancéreuses (cellules HaCaT) et cancéreuses (cellules A431) mutées en p53.

Dans les exemples, sont aussi définis des tests simples pour mettre en évidence la génération d'une situation pro-oxydante, notamment par détection de la production de l'anion superoxyde mitochondrial.

L'huile selon l'invention, peut être extrait des racines, des tiges, de l'écorce ou des feuilles de ladite plante. Des techniques bien maîtrisées sont actuellement connues pour l'extraction des huiles essentielles, notamment l'extraction à froid qui consiste à soumettre la substance végétale à une forte pression à l'aide d'une presse hydraulique, l'entraînement vapeur, qui consiste à former de la vapeur d'eau qui traverse les végétaux et emporte avec elle les molécules aromatiques, et la distillation sèche. L'huile essentielle obtenue par distillation est une essence végétale modifiée par phénomène d'oxydation et d'hydrolyse. Une température maîtrisée et une basse pression sont essentielles pour conserver une qualité aromatique et une composition chimique les plus proches possible de l'essence végétale que l'on cherche à extraire. La plupart des huiles essentielles sont obtenues par distillation et entraînement à la vapeur d'eau (hydro-distillation). D'autres techniques d'extraction sont bien connues et l'homme du métier saura, en fonction de la plante considérée, quelle technique est la plus appropriée. Il saura également, quelle technique est la plus appropriée en fonction de la partie de la plante qui va être utilisée pour l'extraction d'huile essentielle.

Il est bien entendu de préférence fait usage de techniques permettant la meilleure pureté et garantissant l'absence de solvants dans le produit extrait obtenu (huile essentielle ou constituant d'huile essentielle).

Dans le cadre des diverses applications thérapeutiques selon la présente invention, il est de préférence fait usage de compositions comprenant une huile essentielle en combinaison avec d'autres composés. Les autres composés peuvent notamment être des huiles végétales. Parmi les huiles végétales tout particulièrement préférées en combinaison avec les huiles essentielles selon l'invention, ou leurs constituants, on peut citer l'huile de pépins de raisin, l'huile d'amande douce, mais également l'huile de noisette, l'huile de macadamia, l'huile de tournesol et l'huile d'olive. Selon des mises en oeuvre particulières, la composition selon l'invention comprend une huile essentielle en combinaison avec un filtre solaire, ou en combinaison avec une solution hydratante ou une huile végétale, ou bien les deux.

Selon d'autres mises en oeuvre, des compositions selon l'invention, peuvent contenir une combinaison d'au moins deux huiles essentielles distinctes, par exemple une huile essentielle extraite d'une plante du genre Origanum et une huile essentielle extraite d'une plante de la famille des Lauracées.

Il peut également s'agir d'une combinaison comprenant une huile essentielle d'une plante avec un constituant de cette même huile essentielle, ce qui conduit à modifier les proportions naturelles des différents constituants de ladite huile essentielle. Il peut s'agir notamment d'une composition comprenant de l'huile essentielle *d'Origanum compactum* complémentée avec du thymol, du carvacrol et / ou du linalol. Alternativement, il peut s'agir d'une combinaison comprenant une huile essentielle d'une plante du genre Origanum avec un constituant provenant d'une autre huile essentielle, extraite d'une plante différente, notamment de l'huile essentielle *d'Origanum compactum* en association avec du linalol.

Il peut également s'agir d'une composition comprenant deux constituants distincts, l'un présent exclusivement dans l'huile essentielle *d'Origanum compactum* et l'autre présent exclusivement dans l'huile essentielle *d'Aniba rosaeodora.* Par exemple, les deux constituants ainsi choisis ne se trouvent pas naturellement ensemble au sein d'une quelconque huile essentielle, ou bien ne se trouvent pas naturellement dans ces proportions au sein d'une quelconque huile essentielle. D'autres composés additionnels peuvent être choisis en fonction de la texture souhaitée et du mode d'application souhaité, pour les compositions selon l'invention, pour une utilisation dans le traitement ou la prévention des kératoses en phase de transformation, des kératinocytes cancéreux et des carcinomes provenant de la transformation de kératoses.

Par ailleurs, dans la présente invention, les inventeurs ont mis en évidence l'existence d'une gamme spécifique de concentration en huile essentielle *d'Origanum compactum* et *d'Aniba rosaeodora,* ou en constituant de ces deux huiles essentielles, pour laquelle la cytotoxicité est spécifiquement ciblée sur les cellules hyperprolifératives et/ou mutées en p53, par opposition aux cellules normales ou kératinocytes normaux. Les utilisations en thérapie envisagées dans le cadre de la présente invention sont de préférence des utilisations dans la gamme de concentration permettant une cytotoxicité ciblée aux cellules mutées en p53 et assurant une viabilité d'au moins 40%, voire d'au moins 50 ou 60% des cellules normales non mutées. La mise en oeuvre des protocoles détaillés dans les exemples 2 et 3 de la section expérimentale permet à l'homme du métier de déterminer les gammes de concentration adéquates pour toute huile essentielle ou l'un de ses composants.

Dans le cas d'une culture *in vitro,* les inventeurs ont montré (voir exemple 4) que lesdites gammes de concentration sont : de 0.0125% à 0.0175% pour l'huile essentielle d'Origan et de 0.035% à 0.045% pour l'huile essentielle de Bois de rose, afin d'obtenir une cytotoxicité ciblée des cellules mutées en p53.

En fonction de ces données, l'homme du métier saura adapter la composition selon l'invention pour obtenir une gamme de concentration comparable. Il peut notamment adapter la composition selon l'invention sous forme de patch pour garantir une mise en contact prolongée. Il est également envisageable de recommander des applications répétées, par exemple toutes les 24 heures durant une semaine, ou durant un mois, ou plus.

Les diverses utilisations thérapeutiques au sens de l'invention s'entendent d'utilisations dans des conditions telles qu'elles induisent une cytotoxicité préférentielle dans les kératinocytes cancéreux ou pré-cancéreux par rapport aux kératinocytes normaux.

Dans le cadre de cette invention, les utilisations en thérapie mentionnées sont principalement envisagées pour des applications topiques, cutanées. C'est en effet le mode d'administration qui assure non seulement la meilleure efficacité, mais aussi un meilleur ciblage des cellules à traiter.

D'autres modes d'administration sont toutefois envisageables, notamment par voie orale, topique, intra-tumorale, entérale, parentérale, oculaire, sublinguale, inhalée, sous-cutanée, intramusculaire, intraveineuse, transdermique, locale ou rectale. Les formes orales et topiques sont néanmoins préférées. De préférence, la composition pharmaceutique est conditionnée sous une forme convenant à une application par voie topique, par exemple sous forme de patch. Par voie topique, on entend une application sur la peau et/ou les muqueuses.

Une composition utilisée dans le cadre de la présente invention comprend une concentration d'huile essentielle *d'Origanum compactum* d'environ 0,03% à 0,15%, de préférence d'environ 0,03% à 0,1% d'huile essentielle d'Origan, notamment pour une application cutanée.

Les compositions selon l'invention comprennent généralement au moins un excipient pharmaceutiquement acceptable, choisi selon la forme pharmaceutique souhaitée et le mode d'administration choisi. La formulation de la composition est à adapter en fonction du mode d'administration choisi. Pour les formulations à but thérapeutique, on peut envisager les formulations suivantes :
Pour une administration par voie orale, la composition pharmaceutique peut se présenter sous la forme de comprimés, de gélules, de dragées, de sirops, de suspensions, de solutions, de poudres, d'émulsions, de capsules, de microsphères ou nanosphères ou vésicules lipidiques ou polymériques permettant une libération contrôlée.

Par voie parentérale, la composition peut se présenter sous forme de solutions ou suspensions pour perfusion ou pour injection.

Par voie topique, la composition pharmaceutique selon l'invention est plus particulièrement destinée au traitement de la peau et des muqueuses et peut se présenter sous forme d'onguents, de crèmes, de laits, de pommades, de poudres, de tampons imbibés, de solutions, de gels, de sprays, de mousses, de lotions ou de sticks. Elle peut également se présenter sous forme de suspensions de microsphères ou nanosphères ou vésicules lipidiques ou polymériques ou de patches polymériques et d'hydrogels permettant une libération contrôlée. Cette composition par voie topique peut se présenter sous forme anhydre, sous forme aqueuse ou sous la forme d'une émulsion.

Dans le cas des utilisations en prophylaxie, les compositions selon l'invention peuvent être formulées sous forme de lait, par exemple comme lait solaire, sous forme de pommade ou bien sous forme d'huile.

De préférence, l'huile essentielle choisie ou l'un de ses constituants, sera formulé en combinaison avec une huile végétale, notamment pour permettre sa dilution et diminuer ainsi son éventuel effet irritant.

La posologie utile, le nombre d'applications et la durée du traitement varient selon l'âge, le sexe, le poids du patient et le contexte thérapeutique.

La formulation choisie peut être à libération immédiate, prolongée ou retardée.

Par ailleurs, les inventeurs ayant mis en évidence le ciblage spécifique de l'effet cytotoxique des huiles essentielles *d'Origanum compactum* et *d'Aniba rosaeodora* et de leurs constituants, notamment les constituants majoritaires, à l'encontre des cellules hyper prolifératives telles que les cellules mutées en p53, la présente invention concerne également toute utilisation thérapeutique d'une composition comprenant une huile essentielle *d'Origanum compactum* dans le traitement ou la prévention ciblé de zones cancéreuses de la peau dues à l'hyper-prolifération de cellules du derme ou de l'épiderme, par exemple dues à l'hyperprolifération de mélanocytes, notamment dans le cadre de mélanome.

La composition comprenant l'huile essentielle *d'Origanum compactum* est de préférence utilisée en combinaison avec d'autres composés, notamment des excipients thérapeutiquement et/ou pharmaceutiquement acceptables. De tels excipients pharmaceutiquement acceptables sont bien connus de l'homme du métier, il peut s'agir notamment de sucrose, de lactose, d'amidon, de stéarate de magnésium, de sorbitol, de gélatine, de cellulose, de polyéthylène glycol, ou bien d'eau, d'huile ou d'alcool, pour les compositions liquides orales. Cette liste d'excipients utilisables n'est bien entendu pas exhaustive.

De préférence, les compositions décrites précédemment sont pour une utilisation dans le traitement d'un sujet souffrant d'une pathologie liée aux kératoses, notamment un sujet présentant des kératoses en phase de transformation, des kératinocytes précancéreux ou cancéreux ou bien un carcinome issu de kératose. Les carcinomes tout particulièrement considérés dans le cadre de la présente invention sont les carcinomes spinocellulaires (également appelés carcinomes épidermoïdes ou carcinomes squameux), et les carcinomes baso-cellulaires.

Les kératoses, les kératinocytes ou les carcinomes, traités par les compositions de l'invention, se caractérisent par des cellules porteuses de mutations dans la protéine p53. Toutes les cellules considérées ne sont pas nécessairement porteuses de mutation dans la protéine p53, mais de préférence au moins 20% des cellules, au sein de la kératose ou du carcinome à traiter, sont porteuses de mutations dans la protéine p53, de préférence au moins 30%, voire au moins 50% ou plus.

Un sujet susceptible d'être traité par les compositions selon l'invention est un être humain, homme ou femme, quel que soit son âge. De préférence, il s'ait d'un adulte, mais l'invention n'est pas limitée aux adultes et comprend également le traitement d'adolescents, de préférence de plus de 15 ans, par les compositions décrites.

Les compositions mentionnées plus haut sont, de manière particulièrement préférée, pour une utilisation en combinaison ou en association avec un autre traitement contre les kératoses, par exemple en association avec une chimiothérapie, une radiothérapie ou une photothérapie dynamique (P.D.T.). D'autres traitements en combinaison ou en association avec un traitement selon l'invention sont l'électrocoagulation, le laser CO₂, l'application d'azote liquide (cryothérapie), l'application d'une crème qui module l'immunité (imiquimod par exemple), l'application d'une crème à base de fluoro-uracile ou par électrocoagulation. D'autres traitements envisageables concernant les kératoses en phase de transformation, les kératinocytes précancéreux ou cancéreux et les carcinomes issus de kératose sont bien connus de l'homme du métier.

L'alternance entre les différents types de thérapie, la durée de chacune et leur emploi simultané ou séquentiel, seront définis par le médecin au cas par cas, en fonction du patient.

Il est envisagé notamment que la composition selon l'invention soit utilisée pour le traitement des kératoses ou de carcinomes, avant, pendant, après ou en alternance avec un autre type de traitement contre les kératoses ou les carcinomes, notamment un traitement par chimiothérapie , par radiothérapie ou par photothérapie dynamique.

Dans le cadre de la présente invention, il est également envisagé que lesdites compositions soient utilisées pour traiter la peau d'un patient après excision ou ablation d'une kératose en phase de transformation ou présentant un risque de cancérisation. De cette façon, il peut être évité que des cellules non retirées, sur le pourtour de la région excisée, conduisent à la reformation de la kératose ou du carcinome.

Les différents modes de réalisation décrits ci-dessus peuvent bien entendu être combinés entre eux.

La présente divulgation concerne également des compositions pharmaceutiques contenant au moins un des constituants de l'huile essentielle *d'Origanum compactum* ou *d'Aniba rosaeodora,* de préférence une telle composition comprend au moins 10%, ou au moins 15 ou 20%, voire au moins 50% en masse de linalol, de carvacrol ou de thymol.

La divulgation concerne également une composition contenant du linalol, du carvacrol et / ou du thymol pour une utilisation en thérapie, par exemple une composition contenant du linalol et du thymol, ou du carvacrol et du thymol, ou du linalol et du carvacrol, ou bien un mélange des trois, ou bien uniquement l'un des trois. De préférence une telle composition comprend au moins 10%, ou au moins 15 ou 20%, voire au moins 50% en masse de linalol, de carvacrol et/ou de thymol. Les inventeurs ont en effet montré les propriétés thérapeutiques de ces trois composés isolés (voir exemple 3), justifiant leur utilisation comme principe actif dans des applications thérapeutiques.

### Légende des figures :

**FIG.1** **:** La figure 1 représente la mesure de la viabilité cellulaire (test MTT) sur des cellules A431, HaCat et NHEK traitées 4h par l'HE d'Origan. Le trait double (=) correspond à la dose létale à 50% (DL50).
**FIG.2** **:** La figure 2 représente la mesure de la viabilité cellulaire (test MTT) sur des cellules A431, HaCat et NHEK traitées 4h par l'HE de Bois de rose. Le trait double (=) correspond à la dose létale à 50% (DL50).
**FIG.3** : La figure 3 représente la mesure de la viabilité cellulaire (test MTT) sur des cellules A431, HaCat et NHEK traitées 20h par l'HE d'Origan. Le trait = correspond à la dose létale à 50% (DL50).
**FIG.4** : La figure 4 représente la mesure de la viabilité cellulaire (test MTT) sur des cellules A431, HaCat et NHEK traitées 20h par l'HE de Bois de rose. Le trait double (=) correspond à la dose létale à 50% (DL50).
**FIG.5****:** La figure 5 représente la mesure de l'apoptose (test AV/PI), dans les cellules A431, HaCat et NHEK, après 12h de traitement par les HE d'Origan et de Bois de rose.
**FIG.6** : La figure 6 représente la mesure de la fluorescence (activité caspase) par cytomètrie en flux. Les cellules non marquées, les cellules marquées non traitées, et les cellules traitées (par HE) et marquées, sont étiquetées.
**FIG.7** : La figure 7 représente la mesure de la fluorescence (activité caspase) par microscopie à fluorescence.
**FIG.8** **:** La figure 8 illustre la quantification du nombre de cellules A431 et HEK 001 (kératinocytes non mutés) ayant subi une chute du potentiel membranaire mitochondrial après 6h de traitement par les HE d'Origan et de Bois de rose.
**FIG.9** **:** La figure 9 illustre la détection du stress oxydant induit après 4h d'incubation avec les HE d'Origan et de Bois de rose, dans les cellules A431, HaCat et NHEK 894, par la mesure (cytométrie en flux) de la fluorescence la sonde DHR 123.
**FIG.10** : La figure 10 représente la mesure de l'apoptose (test AV/PI), dans les cellules A431 et HEK001 (kératinocytes immortalisés non mutés), après 12h de traitement par les constituants d'huiles essentielles suivants : linalol, carvacrol et thymol.

### Section expérimentale :

### Exemple 1 : Etude analytique des huiles essentielles (HE) d'origan et de bois de rose.

Le choix d'étudier l'HE d'Origan a été déterminé par les travaux du laboratoire du Dr Averbeck qui ont montré une activité anti-génotoxique de cette HE chez la levure (Bakkali, F. et al. 2006). L'HE de Bois de rose a été choisie au regard de ses propriétés bénéfiques sur la peau.

Le présent exemple concerne la caractérisation de la partie volatile de 2 HE bio achetées dans le commerce: une huile essentielle de bois de rose et une huile essentielle d'origan.

La méthodologie appliquée met en jeu, une analyse GC-MS, sur 2 colonnes de polarité différentes (colonne apolaire OV1 et colonne polaire VF WAX).

### Matériel et Méthode

### 1. Matériel

Les analyses ont été réalisées sur des appareils Agilent avec 2 types de colonnes :
- Colonne capillaire apolaire : Colonne capillaire HP-1 Longueur : 50m, diamètre interne : 0.2 mm, film : 0.33 µm.
- Colonne capillaire polaire : Colonne capillaire VF WAX, Longueur 60m, diamètre interne 0.25mm, film : 0.25µm.

Il est important de souligner que tous les pourcentages obtenus sont des pourcentages chromatographiques et non des pourcentages réels de composés présents dans la phase volatile. Pour quantifier exactement les constituants, il faudrait utiliser un étalon interne pour s'affranchir des variations dues à l'appareillage.

2. Identification des ingrédients à partir d'une chromatographie en phase gazeuse sur 2 colonnes de polarités différentes, couplée à un détecteur spectromètre de masse (GC/MS). L'identification des molécules est réalisée à l'aide de bibliothèques de spectres de masse spécifiques aux parfums.

La sensibilité des appareils a permis de quantifier des composés pour lesquels le pourcentage relatif est supérieur ou égal à 0.001%.

La présentation des résultats et la quantification est réalisée, en GC / FID sur colonne apolaire, la quantification des constituants coélués est réalisée sur colonne polaire.

### 3. Echantillons analysés

- HE de bois de rose
- HE d'origan

### Résultats :

### Huile essentielle de Bois de rose :

A la vue des résultats, il apparaît que :
- L'on identifie avec certitude 99,12 % de l'aire totale du chromatogramme.
- 62 molécules ont été identifiées et quantifiées.
- Le tableau 1 de composition (en pourcentages chromatographiques) établi pour l'HE de bois de rose se trouve ci-dessous : 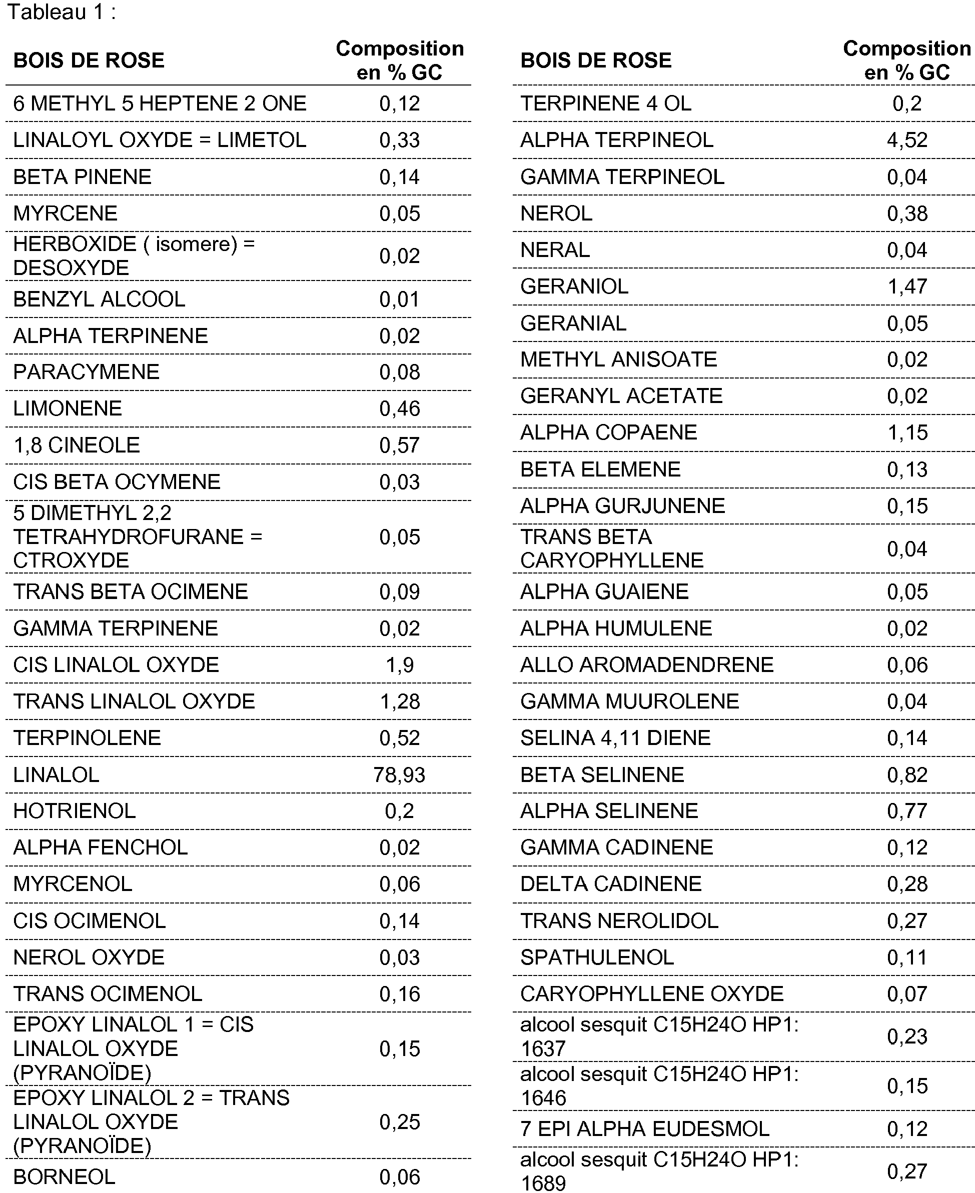 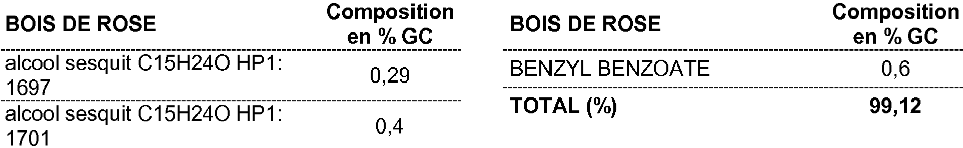
- Le composé majoritaire de l'HE est le linalol (78,93 %).
- Les composés présents en plus grande quantité dans l'HE, après le linalol, sont l'alpha terpinéol (4,52 %), l'oxyde de linalol cis (1,90 %), le géraniol (1,47 %), l'oxyde de linalol trans (1,28 %) et l'alpha copaène (1,15 %).
- Le composé minoritaire est l'alcool benzylique (0,01 %).

Les pourcentages massiques des composés majoritaires de l'huile essentielle de bois de rose sont les suivants :
- Linalol : 78,93%
- Terpinéol alpha : 4,91%.

### Huile essentielle d'origan :

A la vue des résultats, il apparaît que :
- L'on identifie avec certitude 97,69 % de l'aire totale du chromatogramme.
- 20 molécules ont été identifiées et quantifiées.
- Le tableau 2 de composition (en pourcentages chromatographiques) établi pour l'HE d'origan se trouve ci-dessous :

**Tableau 2 :**

| **ORIGAN** | **COMPOSITION EN % GC** |
|---|---|
| Thujène alpha | 0,87 |
| Pinène alpha | 0,64 |
| Camphène | 0,12 |
| 1 octen-3-ol | 0,23 |
| Octanone 3 | 0,17 |
| Myrcène | 1,86 |
| Phellandrène alpha | 0,23 |
| Terpinène alpha | 1,47 |
| Paracymène | 12,65 |
| Phellandrène beta | 0,56 |
| Ocimène trans | 0,07 |
| Terpinène gamma | 15,45 |
| Sabinène trans hydrate | 0,19 |
| Linalol | 1,66 |
| Terpinenol 4 | 0,61 |
| Thymol | 20,23 |
| Carvacrol | 38,61 |
| Caryophyllène beta | 1,88 |
| Humulène alpha | 0,1 |
| Caryophyllène oxyde | 0,09 |
| **TOTAL (%)** | **97,69** |

- Les composés majoritaires de l'HE sont le carvacrol (38,61 %), le thymol (20,23%), le terpinène gamma (15,45 %) et le paracymène (12,65 %).
- Le composé minoritaire est l'ocimène trans (0,07 %).
- Il existe 12 molécules communes entre l'HE d'origan et l'HE de bois de rose: l'alpha pinène, le camphène, le myrcène, l'alpha terpinène, le paracymène, l'ocimène trans, le terpinène gamma, le linalol, le terpinenol 4, le caryophyllène beta, l'alpha humulène, le caryophyllène oyde. Ces composés sont principalement des terpènes.

Les pourcentages massiques des composés majoritaires de l'huile essentielle d'origan sont les suivants :
- Paracymène : 12,27%
- Terpinène gamma : 16,10%,
- Thymol : 18,65%
- Carvacrol : 31,83%.

### Conclusions :

La composition chimique précise de chaque HE a été déterminée par analyse en chromatographie en phase gazeuse (voir tableaux 1 et 2). L'observation de la composition chimique de chaque HE révèle une différence qualitative très nette si l'on considère le nombre de composés majoritaires. Bien que l'HE de Bois de rose présente un plus grand nombre de molécules chimiques différentes (environ 62), elle possède un composé très majoritaire : le linalol, présent à plus de 78% de la fraction analysée. Au contraire, l'HE d'Origan, dans ses 20 molécules recensées, montre quatre molécules majoritaires : le carvacrol (38%), le thymol (20%), le terpinène gamma (15 %) et le paracymène (12 %). A ce titre, l'HE de Bois de rose est dite « mono-moléculaire » et l'HE d'Origan dite « poly-moléculaire ». Il faut également remarquer que l'HE d'Origan est principalement composée de mono-phénol (carvacrol et thymol) alors que l'HE de Bois de rose est constituée majoritairement d'alcool terpénique (linalol).

### Exemple 2 :

Dans le présent travail, la cytotoxicité de deux HE a été plus particulièrement étudiée : l'HE d'Origan compact (*Origanum compactum,* famille des Lamiacées) et l'HE de Bois de rose (*Aniba rosaeodora,* famille des Lauracées) sur des cellules épidermiques humaines : la lignée kératinocytaire HaCat (mutée en p53 et immortalisée spontanément), la lignée A431 (issue d'un carcinome spino-cellulaire) ainsi que sur des kératinocytes épidermiques primaires normaux humains.

Ces trois modèles ont permis d'évaluer les effets biologiques potentiels de ces deux HE sur des cellules épidermiques normales, mutées en p53 (pouvant être assimilées comme précancéreuses) et cancéreuses, l'ensemble représentant dans une certaine mesure, les différentes situations entre peau saine et peau présentant un risque de cancer non mélanome.

### Mesure de la viabilité cellulaire :

Les inventeurs ont comparé l'effet cytotoxique des huiles essentielles sur des kératinocytes normaux, mutés précancéreux (HaCat) et mutés cancéreux (A431).

La viabilité cellulaire est déterminée par le test MTT après un traitement de 4h et 20h par différentes concentrations en HE. Ce test (MTT) est basé sur l'activité enzymatique mitochondriale de la succinate déhydrogénase. Chaque point de viabilité est représenté par trois expériences indépendantes (NHEK : kératinocytes humains normaux « Normal Human Epidermic Keratinocyte »).

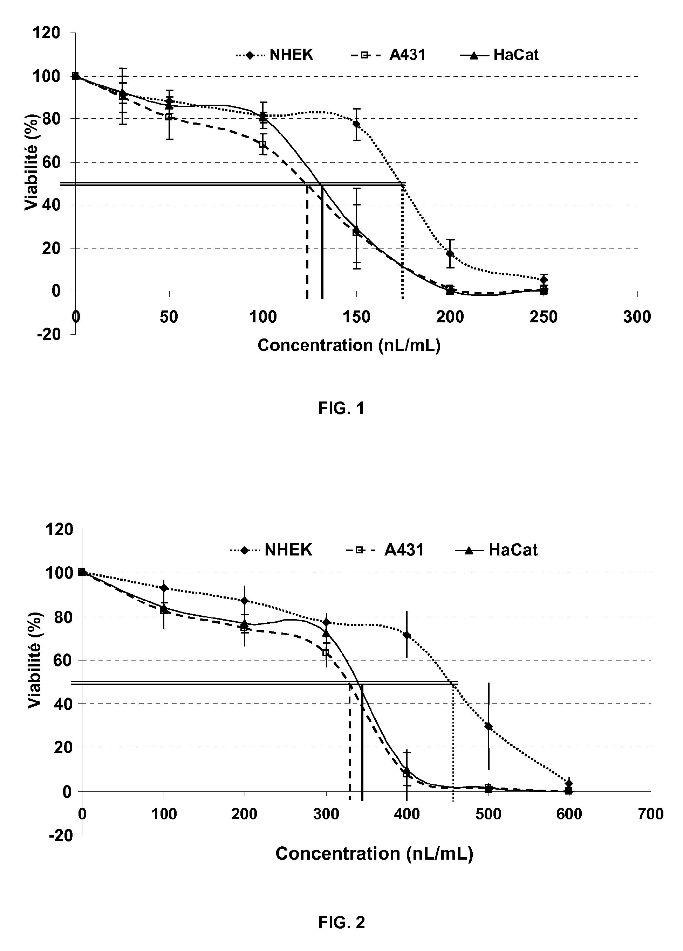

### Les figures 1 et 2 indiquent que :

- l'HE d'Origan et plus cytotoxique que l'HE de Bois de rose, indépendamment du temps (comparaison entre les 4h et 20h de traitement) et de la lignée cellulaire utilisée.
- les cellules normales (NHEK) sont plus résistantes que les cellules cancéreuses (A431) et mutées précancéreuses (HaCat), après 4h de traitement.
- le profil de cytotoxicité est identique entre les cellules A431 et HaCat, après 4h de traitement (par les HE d'Origan et de Bois de rose).

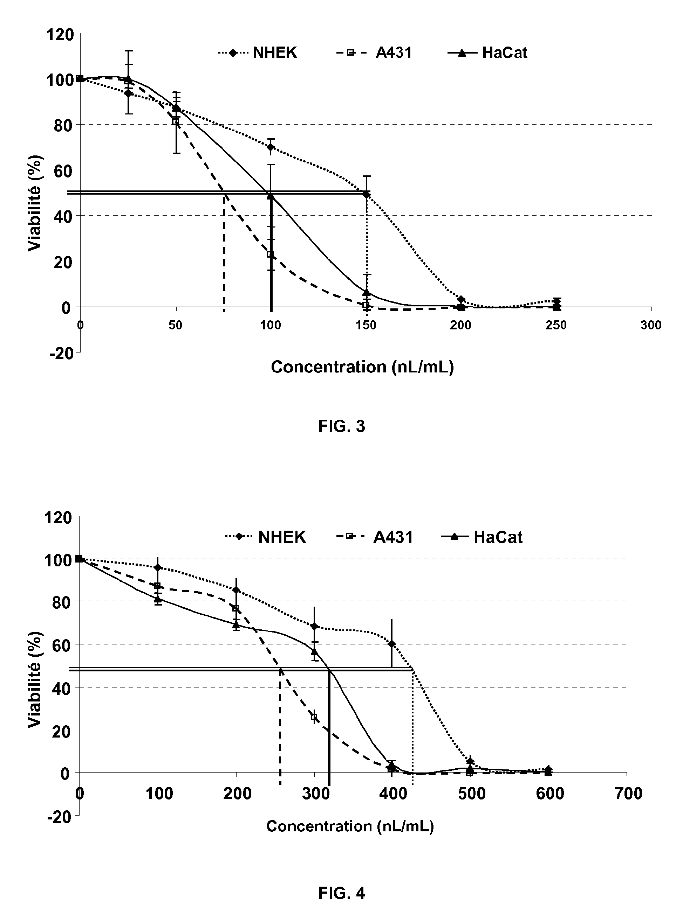

Les figures **3** et **4** confirment que cette différence de toxicité entre les cellules A431/HaCat et les cellules NHEK est conservée après 20h de traitement, bien qu'à ce temps une différence de sensibilité est observée entre les cellules HaCat et A431.

Ces résultats mettent en évidence une toxicité ciblée des HE pour les cellules cancéreuses et mutées précancéreuses par rapport aux cellules normales, à des concentrations de 150 nL/mL et 400 nL/mL d'HE d'Origan et de Bois de rose respectivement.

### Détection de l'apoptose :

L'apoptose se définit par l'induction d'événements biochimiques caractéristiques conduisant à une mort cellulaire programmée. Ces événements caractéristiques sont par exemple, la translocation d'un phospholipide membranaire particulier (phosphatidylserine) de la membrane plasmique interne ou encore l'activation de certaines protéases (caspases) impliquées dans ce processus de dégradation des protéines conduisant à la mort cellulaire.

Les résultats de viabilité cellulaire ont mis en évidence une concentration pour chaque HE, où est révélée la différence de sensibilité des cellules cancéreuses et mutées précancéreuses par rapport aux cellules normales: 150 nL/mL pour l'HE d'Origan et 400 nL/mL pour l'HE de Bois de rose. Ces concentrations seront utilisées dans le cadre d'un traitement de 16h (les 4h et 12h ont également été réalisés).

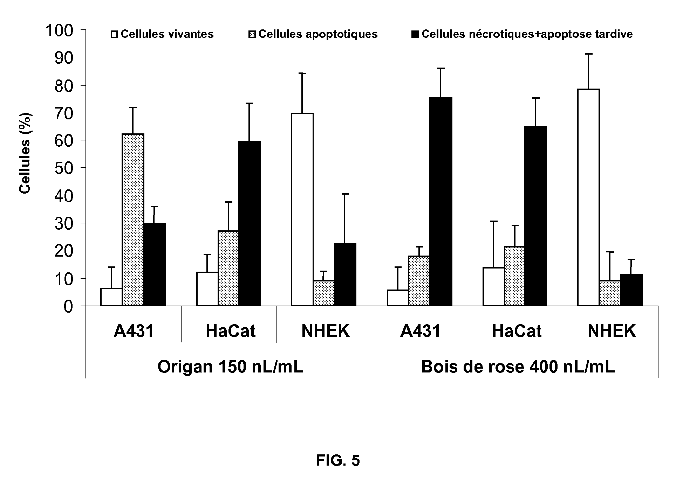

La figure **5** met en évidence après 12h de traitement:
- une viabilité significativement plus élevée des cellules normales (NHEK ; viabilité >70%) par rapport aux lignées cancéreuse A431 et mutée précancéreuse HaCat (taux de viabilité < 15% dans les lignées A431 et HaCat).
- une forte induction de l'apoptose dans la lignée cancéreuse A431 (plus de 60% de cellules annexine V positives).
- une apoptose tardive ou nécrose dans les cellules mutées précancéreuses HaCat.

Ces résultats confirment une sensibilité plus élevée à la toxicité des HE des cellules mutées précancéreuses HaCat et cancéreuses A431 par rapport aux kératinocytes normaux NHEK. Ils mettent également en évidence une induction de mort cellulaire par apoptose.

Afin de confirmer l'induction d'une mort cellulaire par apoptose par les HE, un second test basé sur la détection de l'activité des caspases, une famille de protéines spécifiques du mécanisme de l'apoptose, est mis en oeuvre sur les cellules traitées par les HE. L'activation des caspases est un phénomène plus précoce que la translocation de la phosphatidylsérine détectée précédemment par l'annexine V. Le principe du test est basé sur l'utilisation d'un substrat de ces enzymes couplé à un fluorochrome. Les cellules en apoptose, où les caspases sont activées, dégraderont le substrat et émettent de la fluorescence. Les cellules non apoptotiques n'émettent pas de fluorescence. La mesure de fluorescence est réalisée par cytométrie en flux et microscopie à fluorescence, après 4h de traitement par les HE.

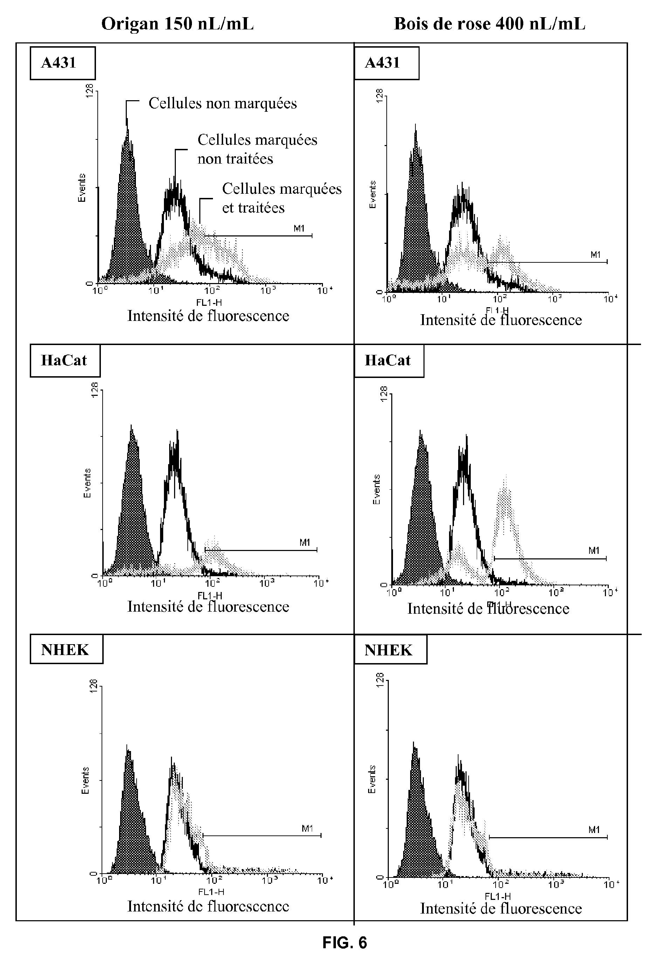

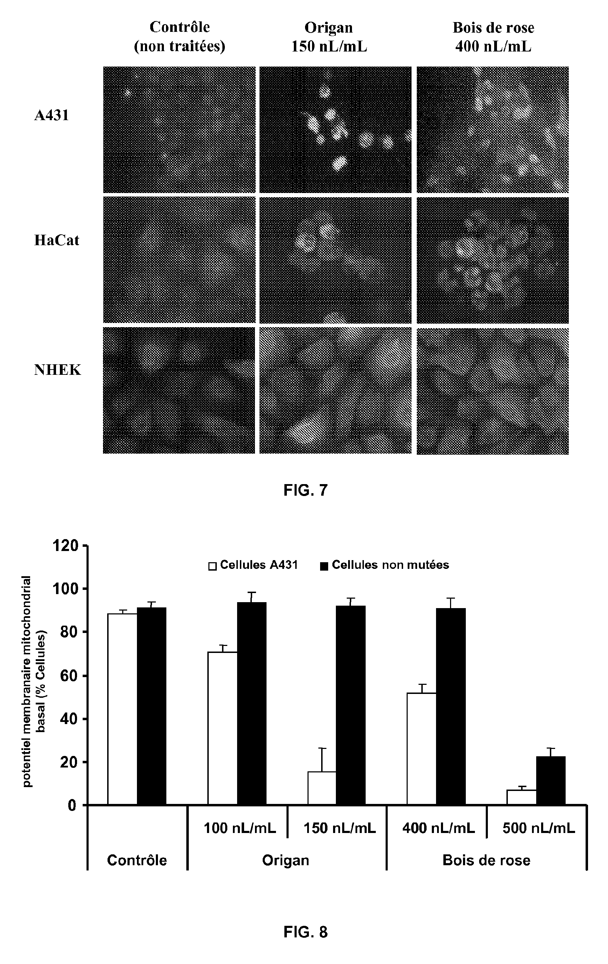

La figure **6** met en évidence un déplacement vers la droite (donc une augmentation de l'intensité de fluorescence) de la courbe représentant lescellules traitées par les HE par rapport à la courbe représentant les cellules marquées mais non traitées, dans les cellules A431 et HaCat uniquement. Cette augmentation de fluorescence, qui est confirmée par microscopie à fluorescence (figure **7**), traduit une activation des caspases et confirme :
- l'induction de l'apoptose dans les cellules cancéreuses, et HaCat par les deux HE, aux concentrations utilisées.
- que cette induction s'observe uniquement dans les cellules A431 et HaCat ; les cellules normales sont donc plus résistantes que les cellules cancéreuses et mutées précancéreuses pour un même traitement par les HE d'Origan et de Bois de rose.

### Effet des HE sur la mitochondrie

Après traitement par les HE d'origan et de bois de rose, l'analyse de l'intégrité de la membrane mitochondriale des kératinocytes mutés cancéreux A431 et des kératinocytes non mutés est réalisée par cytométrie de flux (suivi de la fluorescence liée à l'accumulation de TMRM dans la membrane mitochondriale intègre). On constate en figure **8** que dans le domaine de concentrations choisi, il y a bien un effet déstabilisant de la mitochondrie lié au traitement par les HE, mais cet effet est particulièrement marqué dans les cellules A431 par rapport aux kératinocytes non mutés. Cet impact sur la mitochondrie est très probablement associé au développement du processus apoptotique (cause et/ou conséquence).

### Génération intracellulaire d'espèces réactives de l'oxygène consécutive au traitement par les HE.

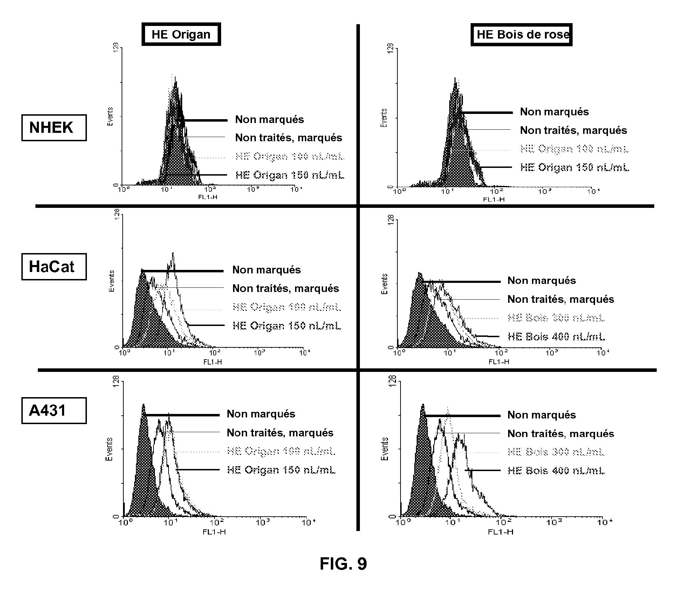

Une des conséquences de la déstabilisation mitochondriale est le relargage de molécules pro-oxydantes dans le cytosol, ce phénomène pouvant être détecté en cytométrie de flux par la sonde DHR123 qui fluoresce en cas de stress oxydant. La figure **9** montre l'évolution du nombre de cellules fluorescentes (donc sujettes à une génération d'espèces réactives de l'oxygène détectables par la DHR123) après différents traitements par les HE. On constate qu'aux concentrations utilisées, les kératinocytes normaux ne sont pas affectés alors qu'une augmentation dose-dépendante du statut pro-oxydant intracellulaire est décelable dans les cellules mutées HaCat ou A431. Ainsi, les HE sont capables de produire un stress oxydant significatif et spécifique dans les kératinocytes mutés précancéreux ou cancéreux, ce stress étant probablement fortement impliqué dans la cytotoxicité ciblée décrite par les figures 1 à 4.

### Conclusions

Les données présentées ici démontrent une sensibilité à la cytotoxicité des HE d'Origan et de Bois de rose significativement plus élevée chez des kératinocytes humains cancéreux et mutés précancéreux par rapport aux kératinocytes normaux.

L'analyse des mécanismes en jeu a permis de montrer que cette différence de sensibilité s'expliquait par l'induction d'une mort par apoptose dans les cellules cancéreuses et mutées précancéreuses, alors que les cellules normales présentaient des taux de viabilité cellulaire supérieurs à 70% pour une même concentration en HE.

L'analyse moléculaire montre que cette toxicité spécifique par les HE implique des modifications de l'intégrité mitochondriale et la génération d'un stress oxydant endogène. Il est donc envisageable de cibler des kératinocytes mutés pré-cancéreux (en particulier dans les kératoses ou dans des zones de la peau endommagées par les UV) par le HE et ainsi de les éliminer spécifiquement.

### Exemple 3 : Mesure de l'activité pro-apoptotique des composés isolés majoritaires des HE d'Origan et de Bois de rose.

L'HE de Bois de rose est composée d'une molécule majoritaire (à plus de 80%) : le linalol (alcool terpénique). L'HE d'Origan Compact contient deux composés majoritaires (mono-terpénol) : le carvacrol et le thymol, présent à 40% et 20%, respectivement.

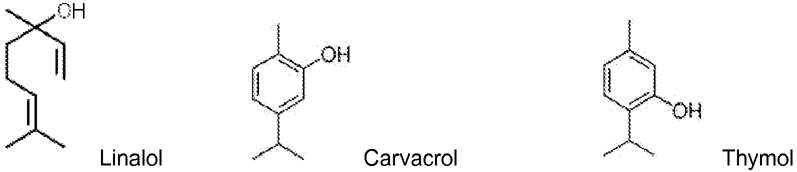

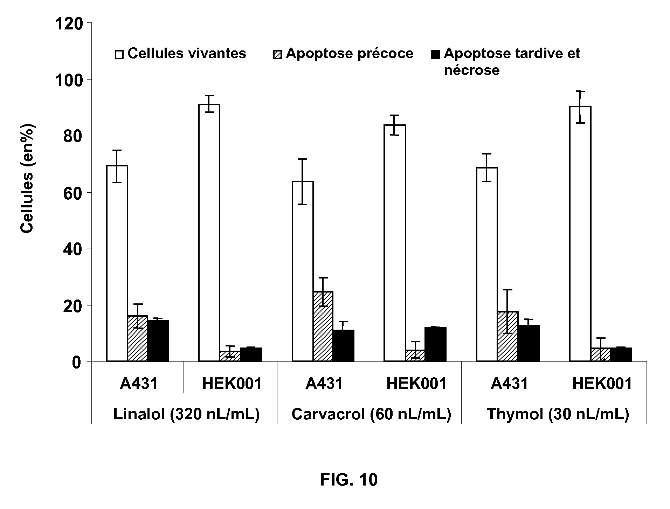

Ces trois molécules, ont été mises en incubation 12h avec des kératinocytes immortalisés non mutés (HEK 001) et des kératinocytes cancéreux mutés (A431). Les pourcentages de cellules en apoptose ont été mesurés par le test Annexine V/ Iodure de propidium. Les concentrations des molécules utilisées sont égales à celles présentes lors des traitements par les HE. Exemple : la concentration de linalol utilisée ici, représente l'équivalent des 80% de linalol présent dans l'HE de Bois de rose. En effet, le linalol est utilisé à une concentration de 320 nL/mL (voir FIG.10), c'est-à-dire 80% de 400 nL/mL d'huile essentielle de Bois de rose qui est la concentration démontrée comme la plus toxique pour les cellules HaCaT et A431, tout en laissant un maximun de cellules NHEK et HEK 001 viables (voir Exemple 2, conclusions sur la section « mesure de la viabilité cellulaire » et FIG. 3 et 4 ainsi que la section « détection de l'apoptose » et FIG.5). Il en est de même pour le carvacrol et le thymol de l'HE d'Origan.

Les résultats d'apoptose sont présentés dans la figure **10****.**

Les résultats de cette expérience exprimés en % de cellules, vivantes, en apoptose ou en apoptose tardive et nécrose montrent:
- Une cytotoxicité et une activité pro-apoptotique plus faible des molécules majoritaires isolées par rapport à celle induite par les HE dans leur complexité (70% d'apoptose induite dans les cellules cancéreuses à dose équivalente d'HE d'Origan, soit 150 nL/mL). En effet, la FIG.5 fait apparaitre par exemple que plus de 60% des cellules A431 sont apoptotiques après un traitement de 12h par de l'HE d'origan à une concentration de 150 nL/mL. La FIG.10 au contraire montre qu'environ 25% seulement des cellules A431 sont apoptotiques après 12h de traitement avec le carvacrol à une concentration de 60nL/mL (correspondant à 40% de 150nL/mL) et environ 15% de cellules apoptotiques avec un traitement au thymol à 30 nL/mL (correspondant à 20% de 150nL/mL).
- Des taux de viabilité des cellules non mutées (HEK001) supérieurs à ceux observés dans les cellules mutées (A431). Le ciblage préférentiel des cellules mutées est donc conservé.

Cette expérience montre que la mort par apoptose ciblée aux cellules cancéreuses mutées est conservée qualitativement si l'on utilise les composés majoritaires du Bois de rose (Linalol) ou de l'Origan (Carvacrol et thymol). Par contre, l'efficacité cytotoxique est moindre qu'avec les HE dans leur complexité. Il existe donc vraisemblablement des synergies avec les autres constituants.

Ce point est confirmé par l'observation réalisée par les inventeurs que le mélange de plusieurs molécules isolées d'une même huile essentielle reproduit l'activité observée avec l'huile essentielle, activité supérieure à la simple addition des activités des molécules isolées prises séparément.

### Matériel et méthodes pour les exemples 2 et 3:

Trente six heures avant le traitement, les cellules sont distribuées en plaque 96 puits (5.10⁵ cellules/mL).

Solution de traitement des HE : les HE sont diluées au 1/10ème une première fois dans de l'éthanol 100%. Puis une seconde dilution est réalisée dans du milieu de culture afin d'obtenir les concentrations désirées.

La mesure de viabilité est réalisée à l'aide du test MTT (1-(4,5-Dimethylthiazol-2-yl)-3,5-diphenylformazan. Sigma-Aldrich). Brièvement, après le temps de traitement désiré, le milieu de traitement est remplacé par du milieu contenant la solution de MTT (0,25 µg/mL de concentration finale). La plaque 96 puits contenant les cellules est ensuite incubée 4h à 37°C avant d'éliminer la solution de MTT et d'ajouter 100 µL de DMSO. La mesure d'absorbance est ensuite réalisée à 540 nm après avoir homogénéisé le précipité violet.

La mesure du stress oxydant est réalisée à l'aide de la sonde fluorescente DHR 123 (Molecular Probe). Après 4h de traitement par les HE, le milieu est éliminé et les cellules sont récoltées (par trypsinisation), centrifugées puis rincées dans 4mL de PBS 1X. Les cellules sont ensuite incubées 30 min (37°C) dans du milieu de culture contenant la sonde (5µM). Avant la mesure de fluorescence par cytométrie (Excitation : 488 nm/Emission : 530 nm), les cellules sont rincées dans 4 mL de PBS 1X, centrifugées et reprises dans 0,5 mL de PBS.

### Détection de l'apoptose :

### Test Annexine V/iodure de propidium :

Un des marqueurs de l'apoptose est la translocation du phosphatidyl-serine de la face interne vers la face externe de la membrane plasmique (translocation permettant aux corps apoptotiques d'êtres reconnus et phagocytés par les macrophages).

Le test AV/PI permet de détecter cet événement, grâce à l'affinité de la protéine annexine V pour le phosphatidyl-serine. L'iodure de propidium qui cible l'ADN, permet la discrimination des cellules apoptotiques précoces, des cellules en apoptose tardives et/ou en nécrose.

Après le temps de traitement désiré, le milieu est éliminé, les cellules sont récoltées (par trypsinisation), centrifugées et rincées dans 4 mL de PBS 1X. Le marquage est réalisé en suivant les instructions du fabriquant (Kit Vybrant. Molecular Probe). Rapidement, les cellules sont reprises dans 100 µL de tampon de marquage, dans lequel est ajouté 6µL d'Annexine 5, et 3µg/mL d'iodure de propidium par échantillon. Les cellules sont ensuite incubées 30 min à l'obscurité (à température ambiante). Quatre cent µL de tampon de marquage sont additionnés avant de mesurer la fluorescence de l'annexine 5 (à 530 nm) et de l'iodure de propidium (à 610 nm).

### Activité caspase :

L'activité caspase est un second marqueur de l'apoptose. La famille des caspase regroupe des protéines principalement impliquées dans la régulation de la mort cellulaire par apoptose. L'activité caspase est détectée à l'aide d'un substrat VAD (pour valine-alanine-acide aspartique) couplé à un fluorochrome (FITC. Emission : 530 nm). La détection de cette activité est réalisée en suivant les instructions du kit CasPACE TM (Promega). Après traitement les cellules sont récoltées (par trypsinisation), centrifugées et rincées dans 4ml de PBS 1X. Puis les cellules sont incubées 20 min dans du milieu de culture contenant le réactif (10µM), 20 min à l'obscurité et à température ambiante. Les cellules sont ensuite rincées 2 fois dans 4 mL de PBS 1X avant d'être fixées 30 min dans une solution de formalin. Avant la mesure de fluorescence par cytométrie en flux, les cellules sont rincées 3 fois 5 min) dans du PBS 1X puis reprise dans 0,5 mL de PBS.

### Perméabilisation mitochondriale :

La perméabilisation des pores mitochondriaux est également un marqueur précoce de l'apoptose. Une sonde fluorescente (TMRM : TétraMéthyl Rhodamine Méthyl ester) ; Molecular Probe) se localisant dans la mitochondrie, va diminuer en intensité lorsque celle-ci migre dans le cytoplasme, à la suite de l'ouverture des pores mitochondriaux. Une chute de fluorescence est donc mesurée par cytométrie en flux.

Après 4h de traitement, les cellules sont trypsinées et rincées dans du PBS 1X avant de les incuber 30 min à 37°C dans du milieu de culture contenant la sonde fluorescente (50 nM finale). Après cette incubation, les cellules sont rincées 1 fois 5 min dans du PBS 1X puis reprisent dans 0,5 mL de PBS avant la mesure par cytométrie.

### Exemple 4 : Gamme de concentration en HE : cytotoxicité ciblée

Les gammes de concentrations en HE (en % d'HE par volume de milieu de culture) pour lesquelles on observe la propriété de cytotoxicité préférentiellement induite dans les cellules mutées (HaCat et A431) par rapport aux cellules non mutées (HEK001 et NHEK894) sont :
de 0.0125% à 0.0175% d'HE d'Origan.
de 0.035% à 0.045% d'HE de Bois de rose

Dans ces gammes de concentrations, les taux de toxicité sont deux fois élevés dans les cellules mutées que dans les cellules non mutées où la viabilité reste de l'ordre de 60%.

Par ailleurs, il est à souligner que des études ont montré une bonne pénétration cutanée des principaux ingrédients des HE de Bois de rose et d'Origan.

### Références

Bakkali, F., et al. Antigenotoxic effects of three essential oils in diploid yeast (Saccharomyces cerevisiae) after treatments with UVC radiation, 8-MOP plus UVA and MMS. Mutat.Res. 606: 27-38, 2006**.**
Calcabrini, A., et al. Terpinen-4-ol, the main component of Melaleuca alternifolia (tea tree) oil inhibits the in vitro growth of human melanoma cells. J. Invest. Dermatol. 122: 349-360, 2004**.**
Cleaver J. E. and Crowley, E. UV damage, DNA repair and skin carcinogenesis. Front Biosci. 1: d1024-43, 2002**.**
Diaz, C., et al. Chemical composition of Schinus molle essential oil and its cytotoxic activity on tumour cell lines. Nat. Prod. Res. 22: 1521-1534, 2008**.**
Fusenig, N. E. and Boukamp, P. Multiple stages and genetic alterations in immortalization, malignant transformation, and tumor progression of human skin keratinocytes. Mol. Carcinog. 23: 144-158, 1998**.**
Gogvadze V. et al. Mitochondria in cancer cells: what is so special about them ? Trends Cell Biol. 18: 165-173, 2008**.**
Itharat, A., et al. In vitro cytotoxic activity of Thai medicinal plants used traditionally to treat cancer. J.Ethnopharmacol. 90: 33-38, 2004**.**
Kaur, M., et al. Skin cancer chemopreventive agent, α-santalol, induces apoptotic death of human epidermoid carcinoma A431 cells via caspase activation together with dissipation of mitochondrial membrane potential and cytochrome c release. Carcinogenesis 26: 369-380, 2005**.**
Koba K., et al. In vitro cytotoxic activity of Cymbopogon citratus L. and Cymbopogon nardus L. essential oils from Togo. Bangladesh J.Pharmacol. 4: 29-34, 2009**.**
Kulms, D. and Schwarz, T. Molecular mechanisms of UV-induced apoptosis. Photodermatol. Photoimmunol. Photomed. 16: 195-201, 2000**.**
Kumar, A., et al. An essential oil and its major constituent isointermedeol induce apoptosis by increased expression of mitochondrial cytochrome c and apical death receptors in human leukaemia HL-60 cells. Chem. Biol. Interact. 171: 332-347, 2008**.**
Loizzo, M. et al. Antiproliferative effects of essential oils and their major constituents in human renal adenocarcinoma and amelanotic melanoma cells. Cell Prolif. 41: 1002-1012, 2008**.**
Marrot, L. and Meunier, J. R. Skin DNA photodamage and its biological consequences. J. Am. Acad. Dermatol. 58: S139-S148, 2008**.**
Molassiotis, A., et al. Use of complementary and alternative medicine in cancer patients: a European survey. Ann. Oncol. 16: 655-663, 2005**.**
Mudgil, A. V., Segal, N., Andriani, F., Wang, Y., Fusenig, N. E., and Garlick, J. A. Ultraviolet B irradiation induces expansion of intraepithelial tumor cells in a tissue model of early cancer progression. J. Invest. Dermatol. 121: 191-197, 2003**.**
Ortonne, J. P. From actinic keratosis to squamous cell carcinoma. Br. J. Dermatol. 146 Suppl 61: 20-23, 2002**.**
Sharma, P. R., et al. Anticancer activity of an essential oil from Cymbopogon flexuosus. Chem. Biol. Interact. 2008**.**
Taguchi, M., et al. Aberrations of the tumor suppressor p53 gene and p53 protein in solar keratosis in human skin. J. Invest. Dermatol. 103: 500-503, 1994**.**
Verma, M., et al. Induction of mitochondrial-dependent apoptosis by an essential oil from Tanacetum gracile. Planta Med. 74: 515-520, 2008**.**
Wischermann, K., et al. UVA radiation causes DNA strand breaks, chromosomal aberrations and tumorigenic transformation in HaCaT skin keratinocytes. Oncogene 27: 4269-4280, 2008**.**

## Revendications

1. Composition comprenant une huile essentielle extraite de plantes du genre *Origanum* pour une utilisation thérapeutique chez l'être humain dans la prévention, ou le traitement ciblé, de kératoses en phase de transformation cancéreuse, de kératinocytes cancéreux, ou de carcinome provenant de la transformation de kératoses,
lesdites kératoses, kératinocytes cancéreux ou carcinome, **se caractérisant par** des cellules porteuses de mutations dans le gène p53.

2. Composition pour l'utilisation selon la revendication 1, comprenant de l'huile essentielle *d'Origanum compactum.*

3. Composition pour l'utilisation selon la revendication 1, comprenant au moins 15%, de préférence 20% en masse, d'huile essentielle *d'Origanum compactum.*

4. Composition pour l'utilisation selon l'une quelconque des revendications 1 à 3 comprenant d'autres composés, notamment des huiles végétales.

5. Composition pour l'utilisation selon l'une quelconque des revendications 1 à 4 pour une application topique cutanée.

6. Composition pour l'utilisation selon l'une quelconque des revendications 1 à 5, où lesdites kératoses sont des kératoses actiniques.

7. Composition pour l'utilisation selon l'une quelconque des revendications 1 à 5, où ledit carcinome est un carcinome spinocellulaire.

8. Composition pour l'utilisation selon l'une des revendications 1 à 8, où ladite utilisation est en association avec un autre traitement contre les kératoses, notamment en association avec une chimiothérapie ou une photothérapie dynamique (P.D.T.).

9. Composition pour l'utilisation selon l'une quelconque des revendications 1 à 8, où ladite utilisation est avant, pendant, après ou en alternance avec un autre traitement contre les kératoses, notamment un traitement par chimiothérapie ou par photothérapie dynamique.

10. Composition pour l'utilisation selon l'une quelconque des revendications 1 à 10, où ledit traitement ciblé est dépourvu d'effets délétères sur les kératinocytes normaux.

11. Composition pour l'utilisation selon l'une quelconque des revendications 1 à 10, induisant une cytotoxicité préférentielle dans les kératinocytes hyperprolifératifs par rapport aux kératinocytes non-hyperprolifératifs.

12. Composition pour l'utilisation selon l'une quelconque des revendications 1 à 10, où l'huile essentielle constitue un principe actif de la composition.

13. Huile essentielle *d'Origanum compactum* pour une utilisation dans le traitement ou la prévention chez l'être humain, de kératoses en phase de transformation cancéreuse, de kératinocytes cancéreux, ou de carcinome provenant de la transformation de kératoses,
lesdites kératoses, kératinocytes cancéreux ou carcinome, **se caractérisant par** des cellules porteuses de mutations dans le gène p53.

## Patentansprüche

1. Zusammensetzung, umfassend ein aus Pflanzen der Gattung *Origanum* gewonnenes ätherisches Öl für eine therapeutische Anwendung bei einem Menschen in der Prävention oder der gezielten Behandlung von Keratosen in der Krebstransformationsphase, von Keratinozyten-Krebs, oder von aus der Transformation von Keratosen stammendem Karzinom, wobei die Keratosen, der Keratinozyten-Krebs oder das Karzinom sich durch Zellen kennzeichnen, die Mutationen im Gen p53 tragen.

2. Zusammensetzung zur Anwendung nach Anspruch 1, umfassend ätherisches Öl von *Origanum compactum.*

3. Zusammensetzung nach Anspruch 1, umfassend mindestens 15 Gew.-%, bevorzugt 20 Gew.-% ätherisches Öl von *Origanum compactum.*

4. Zusammensetzung zur Anwendung nach einem der Ansprüche 1 bis 3, umfassend andere Verbindungen, insbesondere Pflanzenöle.

5. Zusammensetzung zur Anwendung nach einem der Ansprüche 1 bis 4 für eine topische kutane Anwendung.

6. Zusammensetzung zur Anwendung nach einem der Ansprüche 1 bis 5, wobei die Keratosen aktinische Keratosen sind.

7. Zusammensetzung zur Anwendung nach einem der Ansprüche 1 bis 5, wobei das Karzinom ein Plattenepithelkarzinom ist.

8. Zusammensetzung zur Anwendung nach einem der Ansprüche 1 bis 8, wobei die Anwendung mit einer anderen Behandlung gegen die Keratosen assoziiert ist, insbesondere mit einer Chemotherapie oder einer dynamischen Phototherapie (P.D.T.) assoziiert ist.

9. Zusammensetzung zur Anwendung nach einem der Ansprüche 1 bis 8, wobei die Anwendung vor, während, nach, oder abwechselnd mit einer anderen Behandlung gegen die Keratosen, insbesondere einer Behandlung durch Chemotherapie oder dynamische Phototherapie, stattfindet.

10. Zusammensetzung zur Anwendung nach einem der Ansprüche 1 bis 10, wobei die gezielte Behandlung frei von schädlichen Wirkungen auf normale Keratinozyten ist.

11. Zusammensetzung zur Anwendung nach einem der Ansprüche 1 bis 10, die eine bevorzugte Zytotoxizität in den hyperproliferativen Keratinozyten gegenüber nicht-hyperproliferativen Keratinozyten induziert.

12. Zusammensetzung zur Anwendung nach einem der Ansprüche 1 bis 10, wobei das ätherische Öl einen Wirkstoff der Zusammensetzung darstellt.

13. Ätherisches Öl von *Origanum compactum* für eine Anwendung in der Behandlung oder der Prävention im Menschen, von Keratosen in der Krebstransformationsphase, von Keratinozyten-Krebs, oder von aus der Transformation von Keratosen stammendem Karzinom, wobei die Keratosen, der Keratinozyten-Krebs oder das Karzinom durch Zellen gekennzeichnet sind, die Mutationen im Gen p53 tragen.

## Claims

1. A composition comprising an essential oil extracted from plants of the genus *Origanum,* for therapeutic use in human beings in the prevention or the targeted treatment of keratoses in the cancerous transformation phase, of cancerous keratinocytes or of carcinoma originating from the transformation of keratoses,
wherein said keratoses, cancerous keratinocytes or carcinoma are **characterized by** cells carrying mutations in the p53 gene.

2. The composition for use according to claim 1, comprising essential oil of *Origanum compactum.*

3. The composition for use according to claim 1, comprising at least 15%, preferably 20% by weight of the essential oil of *Origanum compactum.*

4. The composition for use according to any one of claims 1 to 3, comprising other compounds, in particular vegetable oils.

5. The composition for use according to any one of claims 1 to 4, for topical application to the skin.

6. The composition for use according to any one of claims 1 to 5, wherein said keratoses are actinic keratoses.

7. The composition for use according to any one of claims 1 to 5, wherein said carcinoma is a spino-cellular carcinoma.

8. The composition for use according to any one of claims 1 to 8, wherein said use is in association with another treatment against keratoses, in particular in association with a chemotherapy or a dynamic phototherapy (DPT).

9. The composition for use according to any one of claims 1 to 8, wherein said use is before, during, after or alternating with another treatment against keratoses, in particular a treatment by chemotherapy or by dynamic phototherapy.

10. The composition for use according to any one of claims 1 to 10, wherein said targeted treatment is free of deleterious effects on normal keratinocytes.

11. The composition for use according to any one of claims 1 to 10, inducing a preferential cytotoxicity in hyperproliferative keratinocytes compared with non-hyperproliferative keratinocytes.

12. The composition for use according to any one of claims 1 to 10, wherein the essential oil constitutes an active principle of the composition.

13. An essential oil of *Origanum compactum* for use in the treatment or the prevention, in the human being, of keratoses in the cancerous transformation phase, of cancerous keratinocytes or of carcinoma originating from the transformation of keratoses,
wherein said keratoses, cancerous kératinocytes or carcinoma are **characterized by** cells carrying mutations in the p53 gene.
